# EUROPEAN PATENT SPECIFICATION

(11) **EP 1 908 390 B1**
(45) Date of publication and mention of the grant of the patent: **23.03.2011**
(21) Application number: 06781415.2
(22) Date of filing: 21.07.2006
(51) Int. Cl.: A61B 1/00, G02B 23/24, A61B 1/005

(54) **ENDOSCOPE**
ENDOSKOP
ENDOSCOPE

(30) Priority: 22.07.2005 JP 2005213055
(43) Date of publication of application: 09.04.2008
(73) Proprietor: OLYMPUS MEDICAL SYSTEMS CORP., Shibuya-ku Tokyo 151-0072 (JP)
(72) Inventor: UENO, Haruhiko c/o IP Support Dptmt. OLYMPUS IP SERVICES CO., LTD., Tokyo 192-8512 (JP); IKEDA, Yuichi c/o IP Support Dptmt. OLYMPUS IP SERVICES CO., LTD., Tokyo 192-8512 (JP); SATO, Tomoaki c/o IP Support Dptmt. OLYMPUS IP SERVICES CO., LTD., Tokyo 192-8512 (JP); NAKAMURA, Shuji c/o IP Support Dptmt. OLYMPUS IP SERVICES CO., LTD., Tokyo 192-8512 (JP)
(74) Representative: von Hellfeld, Axel
(86) International application number: PCT/JP2006/314493
(87) International publication number: WO 2007/011028

(56) References cited:
- JP-A- 04 197 235
- JP-A- 2000 014 628
- JP-A- 2000 014 628
- US-A- 4 947 827
- US-A- 5 060 632
- US-A- 5 359 994
- US-A- 5 733 245

## Description

### Technical Field

The present invention relates to an endoscope of a type in which a driving source unit is
attachable/detachable. The endoscope includes a driving source unit which incorporates driving force generating means for bend-operating a bending section of an insertion section of the endoscope, and which is detachably coupled to a proximal end portion of the insertion section of the endoscope via an attachment/detachment section.

### Background Art

Jpn. Pat. Appln. KOKAI Publication No. 2000-014628 (Patent Document 1) discloses an example of a detachable-type endoscope apparatus. This endoscope apparatus is configured such that an insertion section of an endoscope and a proximal-end-side operation section, which is disposed at a proximal end portion of the insertion section, are detachably coupled via an attachment/detachment section. In the insertion section of the endoscope, a bending section, which is bendable, is provided between an elongated flexible portion and a distal end portion. An operation knob of a bend operation mechanism, which bend-operates the bending section, is provided on the operation section side.

In addition, distal end portions of four wire cables for bend-operating the bending section are fixed to a distal end portion of the bending section. Proximal end portions of the wire cables extend toward the proximal end portion of the insertion section. A transmission mechanism for transmitting a driving force, which is transmitted from the operation knob, to the bending section side, is provided on the proximal end side of the insertion section. The transmission mechanism includes guide wheels for reversing the directions of the four wire cables, and driven shafts. The proximal end portions of the wire cables are coupled to the driven shafts via the guide wheels.

A pinion is fixed to a driving shaft of the operation knob of the operation section. The pinion is meshed with a pair of racks which are disposed to be opposed to each other, and driving shafts are coupled to the racks. When the proximal end portion of the insertion section of the endoscope is coupled to the operation section via the attachment/detachment section, the driving shafts and the driven shafts are abutted upon each other. In this state, the bend operation is performed by advancing and retreating the driven shafts.

US 5,060,632 discloses an endoscope apparatus comprising an elongate insert section including an observation window in the distal end portion and inserted into an object to be observed; an observation device for producing an endoscope image, the observation device including a focusing optical system which receives an incident light from the object through the observation window to focus the endoscope image; and a vibration device for vibrating at least a part of the insert section in a direction crossing the axial direction of the insert section, the vibration device including a vibration unit capable of vibrating in itself to vibrate at least the part of the insert section even under a condition that the insert section is not contact with the object.

### Disclosure of Invention

In the structure of the above-described Patent Document 1, the guide wheel for reversing the direction of each wire cable needs to be provided for each wire at the proximal end portion of the insertion section. Hence, a driving force transmission mechanism, in which a plurality of guide wheels are assembled, needs to be provided in the attachment/detachment section between the proximal end portion of the insertion section of the endoscope and the operation section. As a result, the size of the driving force transmission mechanism increases, and there arises the problem that it is difficult to reduce the size of the attachment/detachment section between the proximal end portion of the insertion section of the endoscope and the operation section.

In addition, if the minimum radius of bend of the wire cable is decreased, there is a possibility of breakage of the wire. Hence, it is not possible to make the radius of the guide wheel less than the minimum radius of bend of the wire cable. Furthermore, such a structure is adopted that the proximal end parts, where the directions of wire cables are reversed by guide wheels, are pushed. These factors hinder the reduction in size of the attachment/detachment section between the proximal end portion of the insertion section of the endoscope and the operation section.

The present invention has been made in consideration of the above circumstances, and the object of the invention is to provide an endoscope wherein an attachment/detachment section between a proximal end part of an insertion section and a part that is attached/detached to/from the proximal end part can be reduced in size, and attachment/detachment is made easier between the proximal end part of the insertion section and the part that is
attached/detached to/from the proximal end part.

According to a first aspect of the present invention, there is provided an endoscope according to claim 1. The endoscope may comprise: an insertion section which includes a distal end portion and a proximal end portion and is insertable in a body cavity, the insertion section including a bending section which is constituted by coupling a plurality of bend pieces; a coupling section provided on a side of the proximal end portion of the insertion section; a pair of working shafts provided at the coupling section; a driving source unit which is detachably coupled to the coupling section and includes driving force generating means for generating a driving force for bending the bending section; operation means which is provided in the driving source unit, includes a driving shaft that linearly moves on the basis of the driving force generated by the driving force generating means, and moves at least one of the working shafts at a time of coupling to the coupling section; reversing means which is provided in the coupling section and transmits movement of one of the working shafts, which is moved by the driving shaft, to the other of the working shafts in a direction opposite to a direction of the movement; and bend operation wires each having a distal end portion and a proximal end portion, the distal end portions of the bend operation wires being connected to the bending section, the proximal end portions of the bend operation wires being connected to the working shafts which are mutually reversely moved, the bend operation wires bend-operating the bending section in accordance with the movement of the working shafts.

In the above-described structure, the driving source unit is detachably coupled to the coupling section on the proximal end side of the insertion section including the bending section. When the coupling section and the driving source unit are coupled, the working shafts of the coupling section are engaged with the driving shafts of the operation means of the driving source unit. In this state, the driving shafts of the operation means linearly move on the basis of the driving force that is generated from the driving force generating means of the driving source unit. One of the working shafts is moved in accordance with the linear movement of the driving shafts, and the movement of the one working shaft, which is operated by the driving shafts, is transmitted to the other working shaft via the reversing means in a direction opposite to the direction of the movement of the one working shaft. At this time, the bend operation wire is pulled by the operation of the working shaft that moves toward the driving source unit, and the bending section is bent.

Preferably, the driving source unit includes a pair of the driving shafts which move the pair of working shafts, respectively.

In the above-described structure, when the coupling section and the driving source unit are coupled, the pair of working shafts of the coupling section are engaged with the pair of driving shafts of the operation means of the driving source unit, and the pair of working shafts of the coupling section are moved by the pair of driving shafts of the operation means.

Preferably, the insertion section includes a rigid or flexible insertion tube section having a distal end portion and a proximal end portion, the bending section is configured to be bend-operable in four directions, that is, an upward direction, a downward direction, a leftward direction and a rightward direction, the bend operation wires comprise two wires for an up-and-down bend operation, which bend-operates the bending section in an up-and-down direction, and two wires for a right-and-left bend operation, which bend-operates the bending section in a right-and-left direction, the coupling section includes two sets of the pair of working shafts, and reversing means which is interposed between the pair of working shafts of each of the two sets, and the driving source unit includes: driving source generating means for an up-and-down direction and driving source generating means for a right-and-left direction, which generate driving forces for bending the bending section in the up-and-down direction and the right-and-left direction, respectively; and operation means which includes driving shafts that linearly move on the basis of the driving forces generated by the driving force generating means for the up-and-down direction and the driving source generating means for the right-and-left direction, and move at least one of the working shafts of each of the sets at a time of coupling to the coupling section.

Preferably, the coupling section includes a base plate, the pinion gear is rotatably supported on the base plate, and the base plate includes a guide member which guides the working shafts in a direction of movement.

Preferably, the guide member includes a flat portion for slidable guiding along a side surface of the rack portion provided on the working shaft.

Preferably, the guide member includes rotation prevention means for preventing rotation of the working shaft about an axis thereof.

Preferably, the rotation prevention means is formed by a slit which is extendingly provided in the working shaft on a side opposite to the rack portion along the direction of movement of the working shaft, and a projection portion which is projectingly provided on the guide member and is engaged in the slit.

According to the above-described aspect of the present invention, it is possible to provide an endoscope wherein an attachment/detachment section between a proximal end part of an insertion section and a part that is attached/detached to/from the proximal end part can be reduced in size, and attachment/detachment is made easier between the proximal end part of the insertion section and the part that is attached/detached to/from the proximal end part.

### Brief Description of Drawings

FIG. 1 schematically shows the structure of the entire system of a detachable-type endoscope according to a first embodiment of the present invention;
FIG. 2 is a side view showing the state in which a proximal-end-side coupling section of a scope section of the detachable-type endoscope and a driving source unit are separated in the first embodiment;
FIG. 3 is a longitudinal cross-sectional view of a main part, which shows an internal structure of a large-diameter section of the scope section of the detachable-type endoscope according to the first embodiment;
FIG. 4 is a cross-sectional view taken along line IV-IV in FIG. 3;
FIG. 5 is a cross-sectional view taken along line V-V in FIG. 3;
FIG. 6A is a plan view showing an arrangement state of driving shafts and working shafts in a case where the bending section of the endoscope of the first embodiment is held in a non-bend state at the time of use;
FIG. 6B is a plan view showing an arrangement state of the driving shafts and working shafts in a case where the bending section is bend-operated;
FIG. 7 is a longitudinal cross-sectional view of a main part, which shows an internal structure of a large-diameter section of the scope section of an endoscope according to a second embodiment of the invention;
FIG. 8 is a cross-sectional view taken along line VIII-VIII in FIG. 7;
FIG. 9 is a cross-sectional view taken along line IX-IX in FIG. 7;
FIG. 10 is a side view showing of a main part, which shows an internal structure of a large-diameter section of the scope section of an endoscope according to a third embodiment of the invention;
FIG. 11 schematically shows the structure of the entire system of a detachable-type endoscope according to a fourth embodiment of the present invention;
FIG. 12 is a plan view showing a schematic structure of a main part of a detachable-type endoscope according to a fifth embodiment of the present invention; and
FIG. 13 is a schematic structural view of a main part, which shows an internal structure of a large-diameter section of a scope section of an endoscope according to a sixth embodiment of the invention.

### Best Mode for Carrying Out the Invention

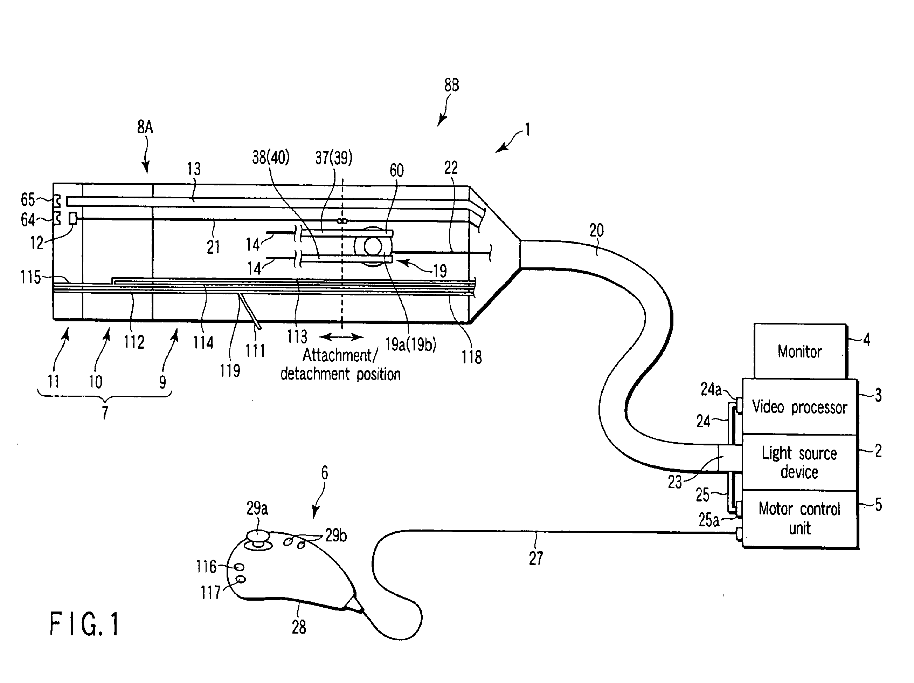

A first embodiment of the present invention will now be described with reference to FIG. 1 to FIG. 6. FIG. 1 schematically shows the structure of the entire system of an endoscope according to the present embodiment. This endoscope system includes a detachable-type endoscope 1, a light source device 2, a video processor 3, a monitor 4, a motor control unit 5, and an operation unit 6 which is an input device for operating the endoscope 1.

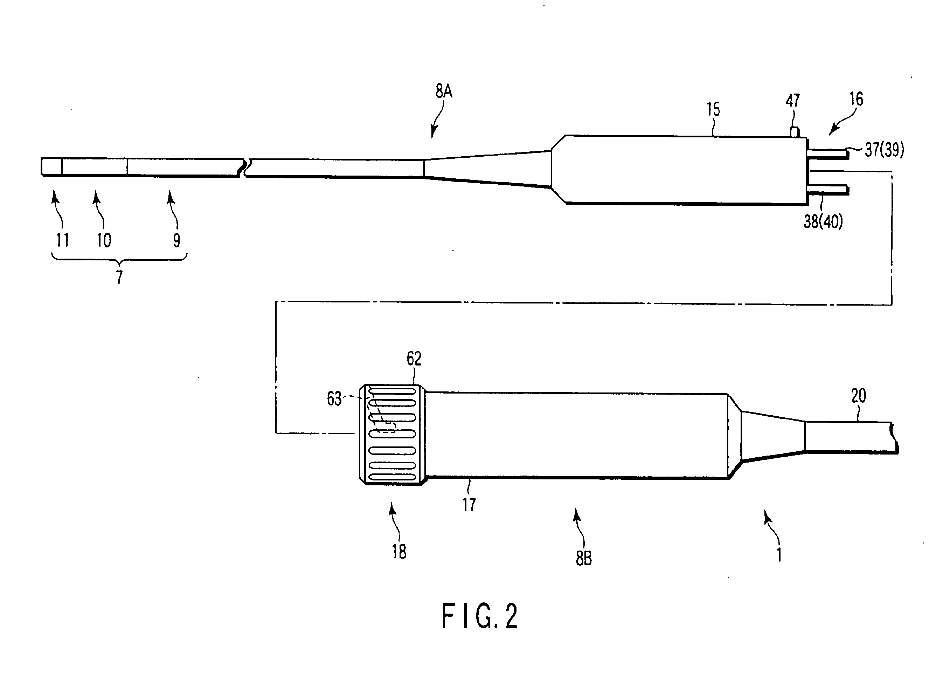

FIG. 2 shows the detachable-type endoscope 1. The detachable-type endoscope 1 comprises a scope section 8A and a driving source unit 8B. The scope section 8A includes an elongated insertion section 7 that is insertable in a body cavity. The driving source unit 8B is detachably coupled to the scope section 8A.

The insertion section 7 of the scope section 8A includes an elongated insertion tube section 9, a bending section 10 that is bendable, and a rigid distal-end structure section 11. The insertion tube section 9 is formed of a rigid tube section such as a metal tube, or a flexible tube section. The bending section 10 is coupled to a distal end of the insertion tube section 9. The distal-end structure section 11 is coupled to a distal end of the bending section 10.

The distal-end structure section 11 incorporates an objective lens 64, an image pick-up device such as a CCD 12 (see FIG. 1), an illumination lens 65, and a distal end portion of a light guide fiber 13. The image pick-up device photoelectrically converts an image which is focused by the objective lens 64. The light guide fiber 13 guides illumination light.

The distal end face of the distal-end structure section 11 is provided with an opening portion of an air/water feed conduit 115 (to be described later) which is built in the insertion section 7, and a distal-end opening portion of a therapeutic device insertion conduit 112 (to be described later). The bending section 10 is configured such that a plurality of substantially ring-shaped bend pieces are juxtaposed in the axial direction of the insertion section 7 and the bend pieces are rotatably coupled via rotational pins such as rivets.

Distal end side portions of four wires 14 for a bend operation are connected to the bending section 10. The four wires 14 bend-operate the bending section 10, for example, in four directions, that is, upward, downward, leftward and rightward directions. Proximal end side portions of the wires 14 are extended toward the proximal end portion of the insertion section 7.

A large-diameter section (coupling section) 15 having a greater diameter than the major part of the insertion tube section 9 is provided on the proximal end side of the insertion tube section 9. A terminal end portion of the large-diameter section 15 is provided with a coupling end section 16 on the scope section 8A side. The coupling end section 16 is detachably coupled to the driving source unit 8B.

A therapeutic device insertion section 111 is projectingly provided on the large-diameter section 15 on the proximal end side of the scope section 8A. A therapeutic device insertion conduit 112 serving also as a suction conduit, a water feed conduit 113 and an air feed conduit 114 are provided within the scope section 8A. A distal end portion of the air feed conduit 114 is connected to a distal end portion of the water feed conduit 113. An air/water feed conduit 115 is formed on the distal end side of the connection part between the water feed conduit 113 and air feed conduit 114. Besides, a proximal end portion of the therapeutic device insertion conduit 112 communicates with the therapeutic device insertion section 111.

The driving source unit 8B is provided with a unit body 17. The unit body 17 has substantially the same diameter as the large-diameter section 15 of the scope section 8A. A coupling end section 18 on the driving source unit 8B side is provided at a distal end portion of the unit body 17. The coupling end section 18 is detachably coupled to the coupling end section 16 of the scope section 8A.

Driving force generating means 19 for generating a driving force for bending the bending section 10 is provided within the unit body 17. The driving force generating means 19 is provided with a driving motor 19a for an up-and-down bend operation and a driving motor 19b for a right-and-left bend operation. The driving motor 19a generates a driving force for bend-operating the bending section 10 in an up-and-down direction. The driving motor 19b generates a driving force for bend-operating the bending section 10 in a right-and-left direction.

A distal end portion of a universal cable 20 is connected to a proximal end portion of the unit body 17 of the driving source unit 8B. A CCD cable 21, a plurality of electrical cables and light guide fiber 13 are contained in the universal cable 20. The CCD cable 21 transmits a video signal from the CCD 12. The plural electrical cables include, for instance, a motor cable 22 for supplying power to the driving motors 19a, 19b of the driving force generating means 19.

A proximal end portion of the universal cable 20 is provided with a connector 23. The connector 23 is detachably connected to the light source device 2. When the light source device 2 and the connector 23 are coupled, illumination light, which is emitted from the light source device 2, is guided to the scope section 8A via the light guide fiber 13.

One end of a video cable 24 and one end of a motor cable 25 are connected to the connector 23. The other end of the video cable 24 is connected to the CCD cable 21. The other end of the motor cable 25 is connected to the motor cable 22.

The said one end of the video cable 24 and the said one end of the motor cable 25 are further extended to the outside from the connector 23. A video connector 24a is connected to an extended end portion of the video cable 24. Similarly, an electrical connector 25a is connected to an extended end portion of the motor cable 25. The video cable 24 is detachably connected to the video processor 3 via the video connector 24a, and the motor cable 25 is detachably connected to the motor control unit 5 via the electrical connector 25a.

The video processor 3 is connected to the monitor 4. At the time of endoscopic observation, an observation image of the scope section 8A, which is captured by the CCD 12, is output in the state in which the observation image is converted to an electric signal. The output signal is input to the video processor 3 via the CCD cable 21 and video cable 24. Further, the above-mentioned output signal is signal-processed by the video processor 3, and is then sent to the monitor 4. The observation image of the scope section 8A is displayed on the screen of the monitor 4.

The operation unit 6 for operating the endoscope 1 is connected to the motor control unit 5 via a cable 27. The operation unit 6 includes a handpiece 28 which can be operated by one hand of the user, almost like a mouse for a personal computer. The handpiece 28 is provided with a joystick 29a for remotely bend-operating the bending section 10, an air/water feed operation button 116, a suction button 117, and other plural remote switches 29b.

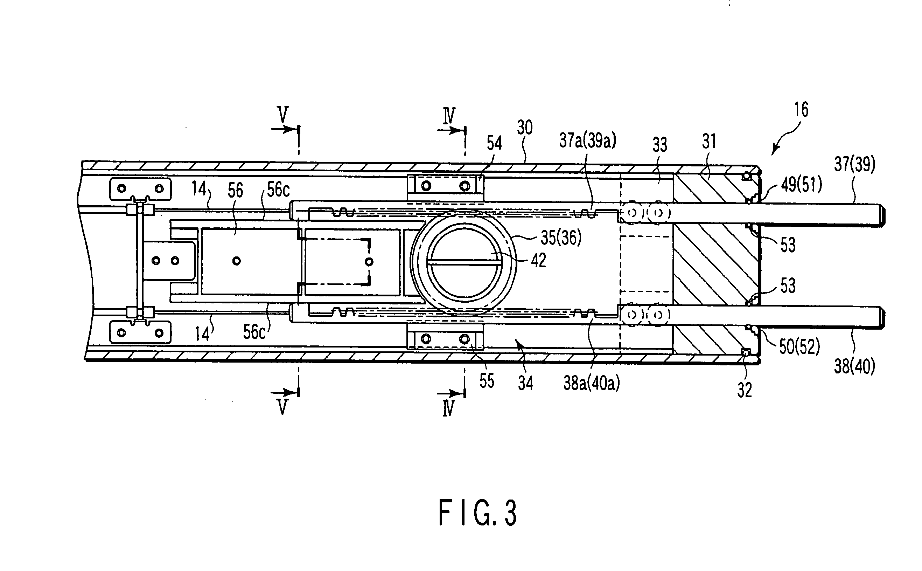

FIG. 3 shows the internal structure of the large-diameter section 15 of the scope section 8A. The large-diameter section 15 of the scope section 8A is provided with a cylindrical cover 30, and a disc-shaped end plate 31. The end plate 31 is fixed in the state in which the end plate 31 closes a terminal-end-side opening portion of the cover 30. An O-ring 32 is provided between the outer peripheral surface of the end plate 31 and the inner peripheral surface of the terminal end portion of the cover 30. Water-tight sealing between the outer peripheral surface of the end plate 31 and the inner peripheral surface of the terminal end portion of the cover 30 is effected by the O-ring 32.

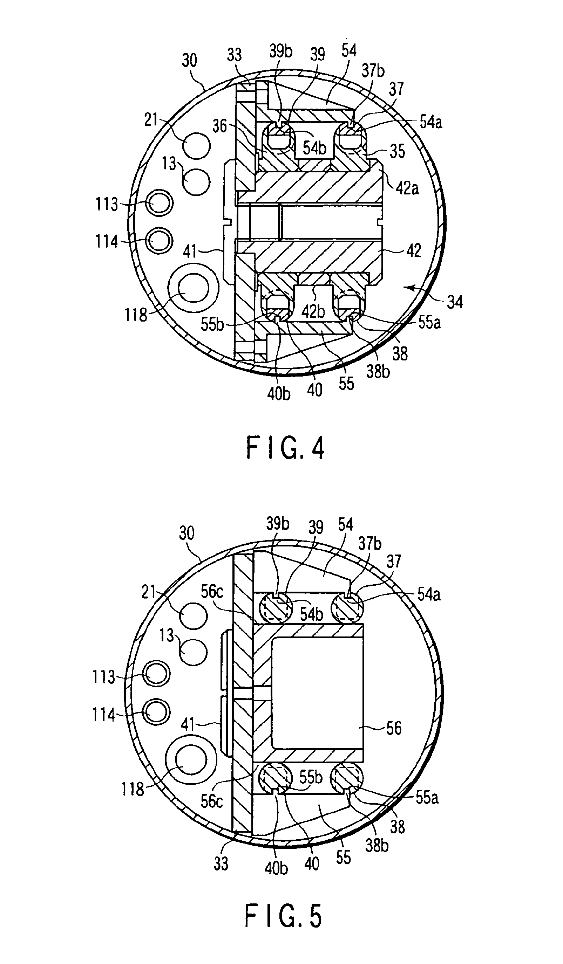

A base plate 33 is provided within the cover 30. As shown in FIG. 4, the base plate 33 is provided to extend over the entire length of the large-diameter section 15. The inside of the cover 30 is substantially partitioned into two spaces by the base plate 33. One end portion of the base plate 33 is fixed to the end plate 31 by fixing screws (not shown).

In the large-diameter section 15 of the scope section 8A, driving force transmission means 34 is built. The driving force transmission means 34 is disposed in the right-side space of the base plate 33 in FIG. 4. The driving force transmission means 34 includes two (first and second) pinion gears 35 and 36 and four working shafts 37, 38, 39 and 40. The driving force transmission means 34 transmits a driving force for the bending section 10, which is supplied from the driving source unit 8B side, as a pulling force of the wires 14 for the bend operation.

One pinion shaft 42 is projectingly provided on the base plate 33. A leg portion of the pinion shaft 42 is fixed to the base plate 33 by a fixing screw 41. A flange-shaped gear holder 42a is projectingly provided at a head portion of the pinion shaft 42. Further, the first and second pinion gears 35 and 36 are mounted on the outer peripheral surface of the pinion shaft 42. An annular spacer 42b is provided between the first and second pinion gears 35 and 36. The first pinion gear 35 and second pinion gear 36 are juxtaposed on the pinion shaft 42 in the state in which the first pinion gear 35 and second pinion gear 36 are spaced apart and mutually opposed via the spacer 42b. Thereby, the first pinion gear 35 and second pinion gear 36 are independently rotatably journaled on the pinion shaft 42.

A pair of working shafts 37 and 38 for the up-and-down bend operation are disposed in parallel and opposed to each other on both sides of the first pinion gear 35 (i.e. upper and lower sides in FIG. 3 and FIG. 4). The working shafts 37 and 38 are provided with rack portions 37a and 38a which are meshed with the first pinion gear 35. Each working shaft 37, 38 is not provided with the rack portion 37a, 38a over about half the length of the working shaft 37, 38 on the proximal end portion side thereof (on the end plate 31 side), and that part of the working shaft 37, 38, which is not provided with the rack portion 37a, 38a, has a circular cross section. The distal-end-side portion of the working shaft 37, 38 is also provided with a shaft portion having a circular cross section, on which the rack portion 37a, 38a is not provided.

The first pinion gear 35, which is interposed between the rack portions 37a and 38a of the working shafts 37 and 38, constitutes reversing means which transmits movement of one working shaft 37 (or 38) to the other working shaft 38 (or 37), with the direction of the movement being reversed.

A pair of working shafts 39 and 40 for the right-and-left bend operation are disposed in parallel and opposed to each other on both sides of the second pinion gear 36 (i.e. upper and lower sides of the second pinion gear 36 in FIG. 3 and FIG. 4). The working shafts 39 and 40 are provided with rack portions 39a and 40a which are meshed with the second pinion gear 36. Each working shaft 39, 40 is not provided with the rack portion 37a, 38a over about half the length of the working shaft 39, 40 on the proximal end portion side thereof (on the end plate 31 side), and that part of the working shaft 39, 40, which is not provided with the rack portion 39a, 40a, has a circular cross section. The distal-end-side portion of the working shaft 39, 40 is also provided with a shaft portion having a circular cross section, on which the rack portion 39a, 40a is not provided.

The second pinion gear 36, which is interposed between the rack portions 39a and 40a of the working shafts 39 and 40, constitutes reversing means which transmits movement of one working shaft 39 (or 40) to the other working shaft 40 (or 39), with the direction of the movement being reversed.

Proximal end portions of the four wires 14 for the bend operation are fixed to distal-end-side end portions of the four working shafts 37, 38, 39 and 40, for example, by means of brazing. For example, proximal end portions of the two wires 14 for bend-operating the bending section 10 in the up-and-down direction are fixed to the pair of working shafts 37 and 38 for the up-and-down bend operation. Similarly, proximal end portions of the two wires 14 for bend-operating the bending section 10 in the right-and-left direction are fixed to the pair of working shafts 39 and 40 for the right-and-left bend operation.

Four passage holes 49, 50, 51 and 52 are formed in the end plate 31. The four passage holes 49, 50, 51 and 52 pass the shaft portions with circular cross sections of the four working shafts 37 to 40. The four working shafts 37 to 40 are passed through the passage holes 49, 50, 51 and 52 so as to be linearly movable in the axial direction of the insertion section 7.

Proximal-end-side shaft end portions of the four working shafts 37 to 40 are held in the state in which these proximal-end-side shaft end portions project to the outside of the large-diameter section 15 from the passage holes 49, 50, 51 and 52 of the end plate 31. Thereby, as shown in FIG. 2, the proximal-end-side shaft end portions of the four working shafts 37 to 40 are held in the projecting state at the coupling end section 16 on the scope section 8A side. An engagement pin 47 (to be described later) for an attachment/detachment mechanism is projectingly provided on the outer peripheral surface of the proximal end portion of the large-diameter section 15 at the coupling end section 16 on the scope section 8A side.

O-rings 53 are fitted at engagement portions between the four passage holes 49, 50, 51 and 52 of the end plate 31 and the shaft portions with circular cross sections of the working shafts 37 to 40. The O-rings 53 effect water-tight sealing of the engagement portions between the four passage holes 49, 50, 51 and 52 of the end plate 31 and the working shafts 37 to 40.

Two first guide members 54 and 55 and one second guide member 56 are fixed to the base plate 33 by screws. The two first guide members 54 and 55 guide the linear movement of the working shafts 37 to 40 from the outside of the working shafts 37 to 40, and the second guide member 56 guides the linear movement from the inside.

The first guide member 54, which is one of the two first guide members 54 and 55, is positioned above the first and second pinion gears 35 and 36 in FIG. 3 and FIG. 4. The other first guide member 55 is positioned below the first and second pinion gears 35 and 36 in FIG. 3 and FIG. 4. The first guide member 54, which is positioned above the first and second pinion gears 35 and 36, and the first guide member 55, which is positioned below the first and second pinion gears 35 and 36, perform positional restriction in a direction in which the shaft portions of the working shafts 37 to 40 move away from the first and second pinion gears 35 and 36.

As shown in FIG. 4, projections 54a and 54b for guiding are projectingly provided on the upper-side first guide member 54 on the side thereof opposed to the working shafts 37 and 39. The projections 54a and 54b are provided to extend in the axial direction of the working shafts 37 and 39. Similarly, projections 55a and 55b for guiding are projectingly provided on the first guide member 55, which is positioned on the lower side in FIG. 3 and FIG. 3, on the side thereof opposed to the working shafts 38 and 40. The projections 55a and 55b are provided to extend in the axial direction of the working shafts 38 and 40.

Slit portions 37b, 38b, 39b and 40b, which extend in the axial direction, are provided in those parts of the outer peripheral surfaces of the working shafts 37 to 40, which are opposite to the rack portions 37a to 40a. The projections 54a and 54b of the first guide member 54, which is positioned on the upper side in FIG. 3 and FIG. 4, are engaged with the slit portions 37b and 39b of the working shafts 37 and 39 so as to be slidable in the axial direction of the working shafts 37 and 39. Similarly, the projections 55a and 55b of the first guide member 55, which is positioned on the lower side in FIG. 3 and FIG. 4, are engaged with the slit portions 38b and 40b of the working shafts 38 and 40 so as to be slidable in the axial direction of the working shafts 38 and 40. Thereby, prevention of rotation of each working shaft, 37 to 40 about the axis thereof, and positional restriction in the right-and-left direction in FIG. 4 are achieved by engagement parts between the projections 54a and 54b of the first guide member 54 and the slit portions 37b and 39b of the working shafts 37 and 39 and engagement parts between the projections 55a and 55b of the first guide member 55 and the slit portions 38b and 40b of the working shafts 38 and 40.

The slit portions 37b to 40b of the working shafts 37 to 40 are not provided over the entire lengths of the working shafts 37 to 40, but are provided only on those portions that are substantially opposed to the rack portions 37a to 40a.

As shown in FIG. 5, the second guide member 56 is disposed inside the working shafts 37 to 40. The second guide member 56 is provided with side surface portions 56c. The side surface portions 56c are put in contact with pinion-side outer peripheral parts of the distal-end-side circular cross-sectional portions of the working shafts 37 to 40, and guide them. The side surface portions 56c restrict the shaft portions of the working shafts 37 to 40 so as not to approach the first and second pinion gears 35 and 36 more closely than necessary.

In the large-diameter section 15 of the scope section 8A, the light guide fiber 13, CCD cable 21, water feed conduit 113, air feed conduit 114 and suction conduit 118, which are contained in the insertion section 7 in the space on the left side of the base plate 33 in FIG. 4, are disposed.

The therapeutic device insertion conduit 112 passes a therapeutic device, which is inserted via the therapeutic device insertion section 111. The therapeutic device insertion conduit 112 is also used as a passage for sucked matter at the time of sucking. The therapeutic device insertion conduit 112 is connected to the separate suction conduit 118 via a branch portion 119. Sucked matter can be sucked into the suction conduit 118 from the therapeutic device insertion conduit 112 via the branch portion 119.

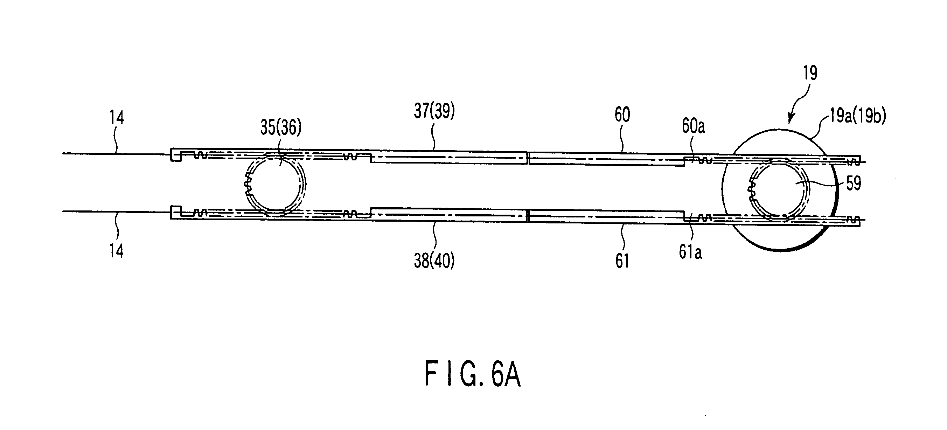

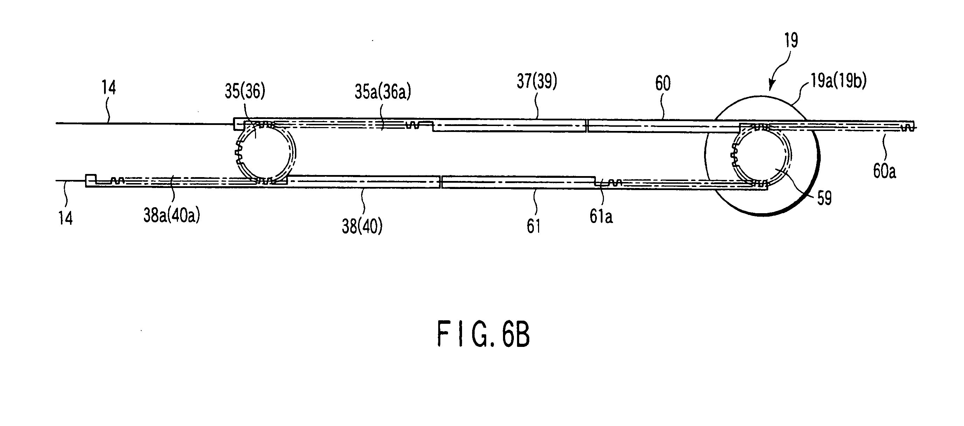

As shown in FIG. 6A and FIG. 6B, the driving source unit 8B is provided with the first driving motor 19a and the second driving motor 19b. The first driving motor 19a serves as the driving source for an up-and-down bend operation and the second driving motor 19b serves as the driving source for a right-and-left bend operation. In the present embodiment, bending in the four directions is exemplified. However, the operation means for the up-and-down bend operation and the operation means for the right-and left bend operation, which are built in the driving source unit 8B, have the same structure. Thus, the structure of only the operation means for the up-and-down bend operation will be described below, and a description of the operation means for the right-and-left bend operation is omitted.

The operation means for the up-and-down bend operation includes a driving pinion 59 which is provided on the rotational shaft of the driving motor 19a, and a pair of driving shafts 60 and 61. The pair of driving shafts 60 and 61 are disposed to be opposed in parallel on both sides of the driving pinion 59 (i.e. on upper and lower sides of the driving pinion 59 in FIG. 6A, 6B). The driving shafts 60 and 61 are provided with rack portions 60a and 61a which are meshed with the driving pinion 59.

As shown in FIG. 2, a lock ring 62 is provided on the coupling end section 18 of the driving source unit 8B. The lock ring 62 is detachably coupled to the coupling end section 16 of the scope section 8A. The lock ring 62 is supported on the coupling end section 18 of the driving source unit 8B so as to be rotatable about the axis thereof.

A cam groove 63, for instance, is formed in an inner peripheral surface of the lock ring 62. The cam groove 63 is disengageably engaged with the engagement pin 47 of the coupling end section 16 of the scope section 8A. When the scope section 8A and the driving source unit 8B are to be coupled, the coupling end section 16 of the scope section 8A and the coupling end section 18 of the driving source unit 8B are abutted upon each other. At this time, the engagement pin 47 on the scope section 8A side is inserted in and engaged with the cam groove 63 of the driving source unit 8B. In this state, the lock ring 62 is rotated over a desired rotational angle. Thereby, the engagement pin 47 is moved to the lock position at the terminal end of the cam groove 63, and the scope section 8A and the driving source unit 8B are locked in the coupled state.

When the scope section 8A and the driving source unit 8B are to be coupled, the end faces of the proximal-end-side portions of the paired working shafts 37 and 38 for the up-and-down bend operation on the scope section 8A side are abutted upon the end faces of the distal-end-side portions of the driving shafts 60 and 61 for the up-and-down bend operation of the driving source unit 8B. At the same time, the end faces of the proximal-end-side portions of the paired working shafts 39 and 40 for the right-and-left bend operation are set to be abutted upon the end faces of the distal-end-side portions of the driving shafts 60 and 61 for the right-and-left bend operation of the driving source unit 8B. In this state, the working shafts 37 and 38 advance and retreat in interlock with the advancement/retreat of the driving shafts 60 and 61 for the up-and-down bend operation of the driving source unit 8B, and thus the bending section 10 is bend-operated in the up-and-down direction. In addition, the working shafts 39 and 40 advance and retreat in interlock with the advancement/retreat of the driving shafts 60 and 61 for the right-and-left bend operation of the driving source unit 8B, and thus the bending section 10 is bend-operated in the right-and-left direction.

Connection parts of the light guide fiber 13, CCD cable 21, water feed conduit 113, air feed conduit 114 and suction conduit 118, which are contained in the insertion section 7, are provided at the coupling section between the coupling end section 18 of the driving source unit 8B and the coupling end section 16 of the scope section 8A. When the scope section 8A and the driving source unit 8B are to be coupled, the connection parts of the light guide fiber 13, CCD cable 21, water feed conduit 113, air feed conduit 114 and suction conduit 118 on the scope section 8A side are detachably connected to the connection parts of the light guide fiber 13, CCD cable 21, water feed conduit 113, air feed conduit 114 and suction conduit 118 on the driving source unit 8B side.

Next, the operation of the above-described structure is described. When the detachable-type endoscope 1 of this embodiment is used, the scope section 8A and the driving source unit 8B are coupled and used. At the time of the work of coupling the scope section 8A and the driving source unit 8B, the coupling end section 16 of the scope section 8A and the coupling end section 18 of the driving source unit 8B are abutted upon each other. At this time, the engagement pin 47 on the scope section 8A side is inserted in and engaged with the cam groove 63 of the driving source unit 8B. In this state, the lock ring 62 is rotated over a desired rotational angle.
Thereby, the engagement pin 47 is moved to the lock position at the terminal end of the cam groove 63, and the scope section 8A and the driving source unit 8B are locked in the coupled state.

When the scope section 8A and the driving source unit 8B are to be coupled, the end faces of the proximal-end-side portions of the paired working shafts 37 and 38 for the up-and-down bend operation on the scope section 8A side are abutted upon the end faces of the distal-end-side portions of the driving shafts 60 and 61 for the up-and-down bend operation of the driving source unit 8B. At the same time, the end faces of the proximal-end-side portions of the paired working shafts 39 and 40 for the right-and-left bend operation are set to be abutted upon the end faces of the distal-end-side portions of the driving shafts 60 and 61 for the right-and-left bend operation of the driving source unit 8B.

FIG. 6A shows the initial state of coupling between the scope section 8A and the driving source unit 8B. In this state, the distal-end-side portions of the driving shafts 60 and 61 for the up-and-down bend operation of the driving source unit 8B and the distal-end-side portions of the driving shafts 60 and 61 for the right-and-left bend operation of the driving source unit 8B are held at substantially the same fixed position. At this time, the bending section 10 of the scope section 8A is held in a substantially straight linear shape, without bend.

Further, the connection parts of the light guide fiber 13, CCD cable 21, water feed conduit 113, air feed conduit 114 and suction conduit 118 on the scope section 8A side are detachably connected to the connection parts of the light guide fiber 13, CCD cable 21, water feed conduit 113, air feed conduit 114 and suction conduit 118 on the driving source unit 8B side.

The endoscope 1 is used in the state in which the coupling work between the scope section 8A and the driving source unit 8B is completed and the scope section 8A and the driving source unit 8B are assembled. When the endoscope 1 is used, the movement of the endoscope 1 is controlled by operating the handpiece 28 of the operation unit 6. Specifically, the bending section 10 is remotely bend-operated by operating the joystick 29a of the handpiece 28. The endoscope operations corresponding to the functions of the respective remote switches 29b are performed by operating the remote switches 29b.

When the bending section 10 is bend-operated, the joystick 29a of the handpiece 28 is operated and turned in a desired operation direction. A signal, which occurs in accordance with the turning operation of the joystick 29a, is input to the motor control unit 5. In addition, when the joystick 29a is turned and operated, a control signal corresponding to the turning operation of the joystick 29a is output from the motor control unit 5. Thereby, at least one of the driving motor 19a for the up-and-down bend operation and the driving motor 19b for the right-and-left bend operation, which are provided in the driving source unit 8B, is driven.

In the case where the driving motor 19a for the up-and-down bend operation is driven, the driving pinion 59 of the driving motor 19a is rotated. When the driving pinion 59 is rotated, the paired driving shafts 60 and 61 are advanced/retreated in the axial direction via the meshed portion between the driving pinion 59 and the rack portions 60a and 61a. At this time, the paired driving shafts 60 and 61 are advanced/retreated in opposite directions by the same distance. For example, one driving shaft 60 is advanced by a predetermined distance toward the scope section 8A, and the other driving shaft 61 is retreated in a direction away from the scope section 8A by a distance that is equal to the distance of the advancement of the driving shaft 60.

In addition, the end faces of the proximal-end-side portions of the paired working shafts 37 and 38 for the up-and-down bend operation on the scope section 8A side are abutted upon the end faces of the distal-end-side portions of the driving shafts 60 and 61 for the up-and-down bend operation of the driving source unit 8B. Thus, the paired working shafts 37 and 38 for the up-and-down bend operation on the scope section 8A side advance and retreat in interlock with the advancement/retreat operation of the driving shafts 60 and 61 for the up-and-down bend operation of the driving source unit 8B.

At this time, for example, as shown in FIG. 6B, one working shaft 38 is pushed forward by the driving shaft 61 that advances toward the scope section 8A. Thereby, the working shaft 38 advances toward the distal end of the scope section 8A. In interlock with the advancement operation of the working shaft 38, the first pinion gear 35 rotates clockwise. When the first pinion gear 35 is rotated, the working shaft 37, which is opposed to the working shaft 38, is retreated toward the driving source unit 8B by the distance that is equal to the distance of advancement of the working shaft 38 in interlock with the rotational operation of the first pinion gear 35. At this time, the retreat operation of the working shaft 37 and the advancement operation of the driving shaft 61 are synchronized and performed at the same time. The wire 14, which is positioned on the upper side in FIG. 6B, is pulled by the retreat operation of the working shaft 37, and thus the bending section 10 can be bend-operated in the up-and-down direction.

The wire 14, which is positioned on the lower side in FIG. 6B and is fixed to the working shaft 38 that is pushed forward (to the insertion section 7 side), is pulled by the bending section 10 and drawn to the insertion section 7 side.

When the driving motor 19b for the right-and-left bend operation is driven, the paired driving shafts 60 and 61 for the right-and-left bend operation are advanced/retreated in the axial direction by substantially the same operation. At this time, one driving shaft 60 is advanced by a predetermined distance toward the scope section 8A, and the other driving shaft 61 is retreated in a direction away from the scope section 8A by a distance that is equal to the distance of the advancement of the driving shaft 60.

In addition, the paired working shafts 39 and 40 for the right-and-left bend operation on the scope section 8A side advance and retreat in interlock with the advancement/retreat operation of the driving shafts 60 and 61. At this time, one working shaft 40 is pushed forward and advanced toward the distal end of the scope section 8A by the driving shaft 61 that advances toward the scope section 8A. In interlock with the advancement operation of the working shaft 40, the second pinion gear 36 rotates clockwise. When the second pinion gear 36 is rotated, the working shaft 39, which is opposed to the working shaft 40, is retreated toward the driving source unit 8B by the distance that is equal to the distance of advancement of the working shaft 40 in interlock with the rotational operation of the second pinion gear 36. At this time, the retreat operation of the working shaft 39 and the advancement operation of the driving shaft 61 are synchronized and performed at the same time. The wire 14, which is positioned on the upper side in FIG. 6B, is pulled by the retreat operation of the working shaft 39, and thus the bending section 10 can be bend-operated in the right-and-left direction.

The wire 14, which is positioned on the lower side in FIG. 6B and is fixed to the working shaft 40 that is pushed forward (to the insertion section 7 side), is pulled by the bending section 10 and drawn to the insertion section 7 side.

By the combination of the up-and-down bend operation and the right-and-left bend operation of the bending section 10, the distal-end structure section 11 of the insertion section 7 of the scope section 8A can be bent in a desired direction.

The following advantageous effects can be obtained by the above-described structure. Specifically, according to the present embodiment, there is provided the detachable-type endoscope 1 in which the scope section 8A having the elongated insertion section 7, which is insertable in the body cavity, and the driving source unit 8B are detachably coupled. The driving source unit 8B is provided with two driving motors 19a and 19b for the up-and-down bend operation and right-and-left bend operation, and a pair of driving shafts 60 and 61 which are advanced/retreated in the axial direction in opposite directions by the same distance by the driving motors 19a and 19b. Furthermore, the driving force transmission means 34 that transmits a driving force for the end section 10, which is supplied from the driving source unit 8B side, as a pulling force of the wires 14 for the bend-operation is built in the large-diameter section 15 at the proximal end portion of the scope section 8A. The driving force transmission means 34 includes two (first and second) pinion gears 35 and 36 and four working shafts 37, 38, 39 and 40. The proximal end portions of the wires 14, which are connected to the bending section 10, are fixed to the four working shafts 37, 38, 39 and 40, for example, by means of brazing.

In the present embodiment, when the scope section 8A and the driving source unit 8B are to be coupled, the end faces of the proximal-end-side portions of the paired working shafts 37 and 38 for the up-and-down bend operation on the scope section 8A side are abutted upon the end faces of the distal-end-side portions of the driving shafts 60 and 61 for the up-and-down bend operation of the driving source unit 8B. At the same time, the end faces of the proximal-end-side portions of the paired working shafts 39 and 40 for the right-and-left bend operation are set to be abutted upon the end faces of the distal-end-side portions of the driving shafts 60 and 61 for the right-and-left bend operation of the driving source unit 8B. In this state, when the bending section 10 is bend-operated, the working shaft 38 (or 40), which is pushed toward the insertion section 7 side, rotates the first pinion gear 35 (or second pinion gear 36). As a result, the working shaft 37 (or 39), which is opposed to the working shaft 38 (or 40), is moved toward the driving source unit 8B side. At this time, the wire 14 is pulled by the working shaft 37 (or 39) which moves toward the driving source unit 8B side, and thereby the bending section 10 can be bent.

Thus, in the present embodiment, since the driving force transmission means 34 having the above-described structure is built in the large-diameter section 15 of the scope section 8A, the attachment/detachment section between the coupling end section 16 of the scope section 8A and the coupling end section 18 of the driving source unit 8B can be reduced in size, compared to the prior art, and the coupling end section 16 of the scope section 8A and the coupling end section 18 of the driving source unit 8B can easily be detached/attached.

Moreover, in the present embodiment, since the proximal end portions of the four wires 14 for the bend operation are fixed to the distal-end-side end portions of the four working shafts 37, 38, 39 and 40 which move linearly, there is no need to hold the wires 14 by winding them. When the wires 14 are wound, it is necessary to wind the wires 14 with a certain diameter or more, taking buckling of wires 14 into account. Thus, a space for this is necessary, and the large-diameter section 15 increases in size. In the structure of this embodiment, however, the diameter of the large-diameter section 15 can be decreased. Furthermore, since the proximal end portions of the wires 14 are held in the linear state, a load of repetitive bending at the time of bending does not act on the proximal end portions of the wires 14 and the durability of the wires 14 can be enhanced.

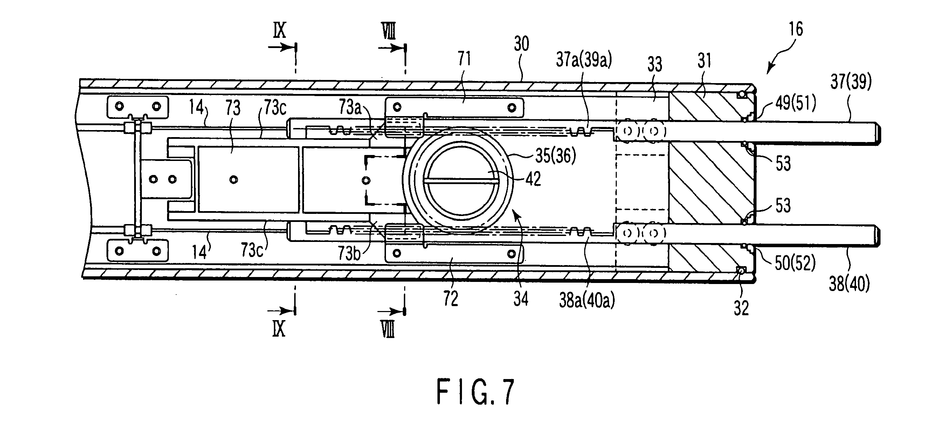

FIG. 7 to FIG. 9 show a second embodiment of the present invention. In this embodiment, the structure of the driving force transmission means 34, which is built in the large-diameter section 15 of the scope section 8A of the detachable-type endoscope 1 according to the first embodiment (see FIG. 1 to FIG. 6B), is altered as described below. The other structural parts are the same as those of the detachable-type endoscope 1 according to the first embodiment. The parts common to those of the detachable-type endoscope 1 according to the first embodiment are denoted by like reference numerals, and a description thereof is omitted.

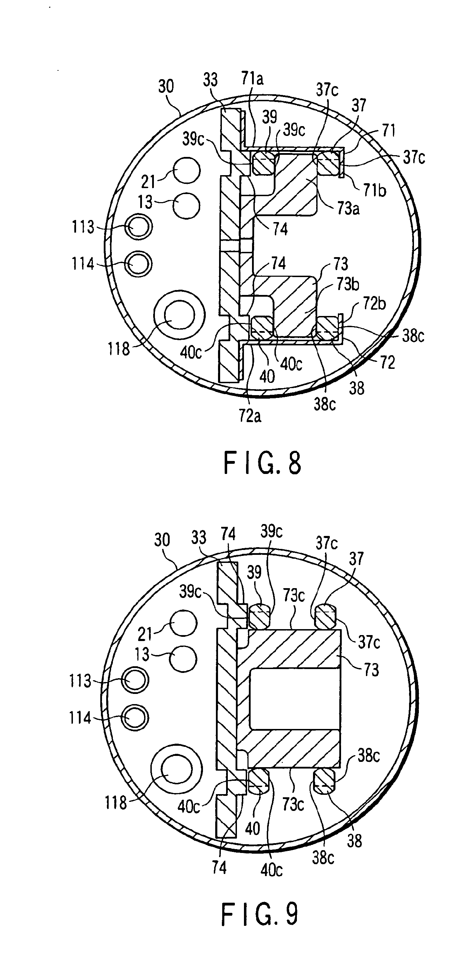

In the driving force transmission means 34 of the present embodiment, as shown in FIG. 8 and FIG. 9, two parallel surfaces 37c, 38c, 39c, 40c, which are parallel to the base plate 33, are provided on both sides of each of four working shafts 37, 38, 39 and 40. The parallel surfaces 37c, 38c, 39c, 40c are formed in the range of the working shaft 37, 38, 39, 40, where the rack portion 37a, 38a, 39a, 40a is provided.

Two third guide members 71 and 72, which guide the linear movement of the working shafts 37 to 40 from the outside of the working shafts 37 to 40, and a fourth guide member 73, which guides the linear movement of the working shafts 37 to 40 from the inside, are fixed to the base plate 33 by screws. The third guide member 71, which is one of the two third guide members 71 and 72, is disposed on the upper side of the two working shafts 37 and 39 in FIG. 8, and the other third guide member 72 is disposed on the lower side of the two working shafts 38 and 40 in FIG. 8.

The upper-side third guide member 71 includes a flat projecting portion 71a and a bent portion 71b.
The flat projecting portion 71a, as shown in FIG. 8, is erected in a direction perpendicular to the base plate 33. The bent portion 71b is bent at a distal end of the projecting portion 71a in an L shape in a downward direction in FIG. 8. The outer parallel surface 37c of the working shaft 37 is put in contact with the inner side surface of the bent portion 71b.

Similarly, the lower-side third guide member 72 includes a flat projecting portion 72a and a bent portion 72b. The flat projecting portion 72a, as shown in FIG. 8, is erected in a direction perpendicular to the base plate 33. The bent portion 72b is bent at a distal end of the projecting portion 72a in an L shape in an upward direction in FIG. 8. The outer parallel surface 38c of the working shaft 38 is put in contact with the inner side surface of the bent portion 72b.

Protruding rail portions 74 are formed on the base plate 33 at positions corresponding to the working shafts 39 and 40. The outer parallel surface 39c of the working shaft 39 is put in contact with the upper-side protruding rail portion 74 in FIG. 8, and the outer parallel surface 40c of the working shaft 40 is put in contact with the lower-side protruding rail portion 74 in FIG. 8.

Further, the fourth guide member 73 is provided with an extension portion 73a and an extension portion 73b. The extension portion 73a extends at a position where the extension portion 73a is inserted between the upper-side two working shafts 37 and 39 in FIG. 8. The extension portion 73b extends at a position where the extension portion 73b is inserted between the lower-side two working shafts 38 and 40 in FIG. 8. The inner parallel surfaces 37c and 39c of the two working shafts 37 and 39 are put in contact with both side surfaces of the upper-side extension portion 73a in FIG. 8. The inner parallel surfaces 38c and 40c of the two working shafts 38 and 40 are put in contact with both side surfaces of the lower-side extension portion 73b in FIG. 8.

Thereby, the two parallel surfaces 37c, 38c, 39c, 40c on both sides of the four working shafts 37, 38, 39, 40 slide along the protruding rail portions 74 of the base plate 33, the bent portions 71b and 72b of the third guide members 71 and 72 and the extension portions 73a, 73b of the fourth guide member 73. Thus, prevention of rotation of each working shaft, 37 to 40, about the axis thereof, and positional restriction in the right-and-left direction in FIG. 8 are achieved.

As shown in FIG. 8, the projecting portion 71a of the upper-side third guide member 71 is disposed so as to come in contact with those outer peripheral parts of the shaft portions of the pair of working shafts 37 and 39, which are opposite to the rack portions 37a and 39a. Similarly, the projecting portion 72a of the lower-side third guide member 72 is disposed so as to come in contact with those outer peripheral parts of the shaft portions of the pair of working shafts 38 and 40, which are opposite to the rack portions 38a and 40a. Thereby, the projecting portion 71a of the upper-side third guide member 71 and the projecting portion 72a of the lower-side third guide member 72 restrict the positions of the shaft portions of the working shafts 37 to 40 in a direction away from the first and second pinion gears 35 and 36.

As shown in FIG. 7 and FIG. 9, like the second guide member 56 shown in FIG. 3, the fourth guide member 73 is provided with side surface portions 73c, which are put in contact with pinion-side outer peripheral parts of the distal-end-side circular cross-sectional portions of the working shafts 37 to 40, and guide them. Thereby, the shaft portions of the working shafts 37 to 40 are restricted so as not to approach the first and second pinion gears 35 and 36 more closely than necessary.

Thus, in the present embodiment, like the first embodiment, since the driving force transmission means 34 having the above-described structure is built in the large-diameter section 15 of the scope section 8A, the attachment/detachment section between the coupling end section 16 of the scope section 8A and the coupling end section 18 of the driving source unit 8B can be reduced in size, compared to the prior art, and the coupling end section 16 of the scope section 8A and the coupling end section 18 of the driving source unit 8B can easily be detached/attached.

Moreover, in the present embodiment, too, since the proximal end portions of the four wires 14 for the bend operation are fixed to the distal-end-side end portions of the four working shafts 37, 38, 39 and 40 which move linearly, there is no need to hold the wires 14 by winding them. When the wires 14 are wound, it is necessary to wind the wires 14 with a certain diameter or more, taking buckling of wires 14 into account. Thus, a space for this is necessary, and the large-diameter section 15 increases in size. In the structure of this embodiment, however, the diameter of the large-diameter section 15 can be decreased. Furthermore, since the proximal end portions of the wires 14 are held in the linear state, a load of repetitive bending at the time of bending does not act on the proximal end portions of the wires 14 and the durability of the wires 14 can be enhanced.

In the present embodiment, in particular, the two parallel surfaces 37c, 38c, 39c, 39d are provided on both side surfaces of the four working shafts 37, 38, 39 and 40, and these parallel surfaces are made to slide along the protruding rail portions 74 of the base plate 33, the bent portions 71b and 72b of the third guide members 71 and 72 and the extension portions 73a, 73b of the fourth guide member 73. Thus, prevention of rotation of each working shaft, 37 to 40, about the axis thereof, and positional restriction in the right-and-left direction in FIG. 8 are achieved. Therefore, the working shafts 37, 38, 39 and 40 can easily be machined, compared to the case where, as in the first embodiment, the working shafts 37 to 40 are provided with the slit portions 37b, 38b, 39b and 40b and the projection portions 54a, 54b of the upper-side first guide member 54 are engaged with the slit portions 37b, 39b of the working shafts 37, 39 and similarly the projection portions 55a, 55b of the lower-side first guide member 55 are engaged with the slit portions 38b, 40b of the working shafts 38, 40.

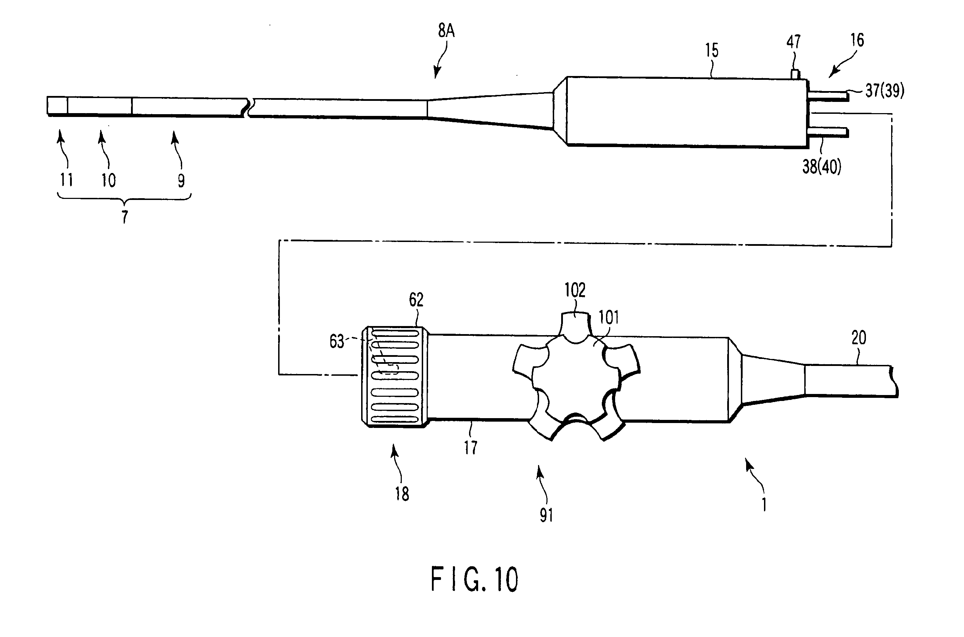

FIG. 10 shows a third embodiment of the present invention. In this embodiment, the motor-type driving force generating means 19, which is provided in the driving source unit 8B of the detachable-type endoscope 1 according to the first embodiment (see FIG. 1 to FIG. 6B), is replaced with manual-type driving force generating means 91. The other structural parts are the same as those of the detachable-type endoscope 1 according to the first embodiment. The parts common to those of the detachable-type endoscope 1 according to the first embodiment are denoted by like reference numerals, and a description thereof is omitted.

Specifically, in the manual-type driving force generating means 91 according to the present embodiment, an operation knob 101 for the up-and-down bend operation and an operation knob 102 for the right-and-left bend operation are provided on a side surface of the driving source unit 8B. The operation knobs 101 and 102 are independently rotatably journaled on the same axis.

The driving source unit 8B contains a bend driving mechanism (not shown) which converts the operation force of the operation knob 101, 102 to linear advancement/retreat movement of the driving shaft 60, 61 in the axial direction.

When the scope section 8A and the driving source unit 8B are coupled, the paired driving shafts 60 and 61 are advanced/retreated in opposite directions by the same distance in accordance with the rotational operation of the operation knob 101, 102 on the scope section 8A side.

The end faces of the proximal-end-side portions of the paired working shafts 37 and 38 for the up-and-down bend operation on the scope section 8A side are abutted upon the end faces of the distal-end-side portions of the driving shafts 60 and 61 for the up-and-down bend operation of the driving source unit 8B. Thus, the paired working shafts 37 and 38 for the up-and-down bend operation on the scope section 8A side advance and retreat in interlock with the advancement/retreat movement of the driving shafts 60 and 61 for the up-and-down bend operation of the driving source unit 8B. At this time, in accordance with the
advancement/retreat movement of each working shaft 37, 38, the wire 14 is pulled toward the driving source unit 8B. With the pulling operation of the wire 14, the bending section 10 is bend-operated.

In the above-described structure, too, the structure of the driving force transmission means 34, which is built in the large-diameter section 15 of the scope section 8A of the detachable-type endoscope 1, is the same as that in the detachable-type endoscope 1 of the first embodiment. Therefore, the same advantageous effects as in the first embodiment can be obtained.

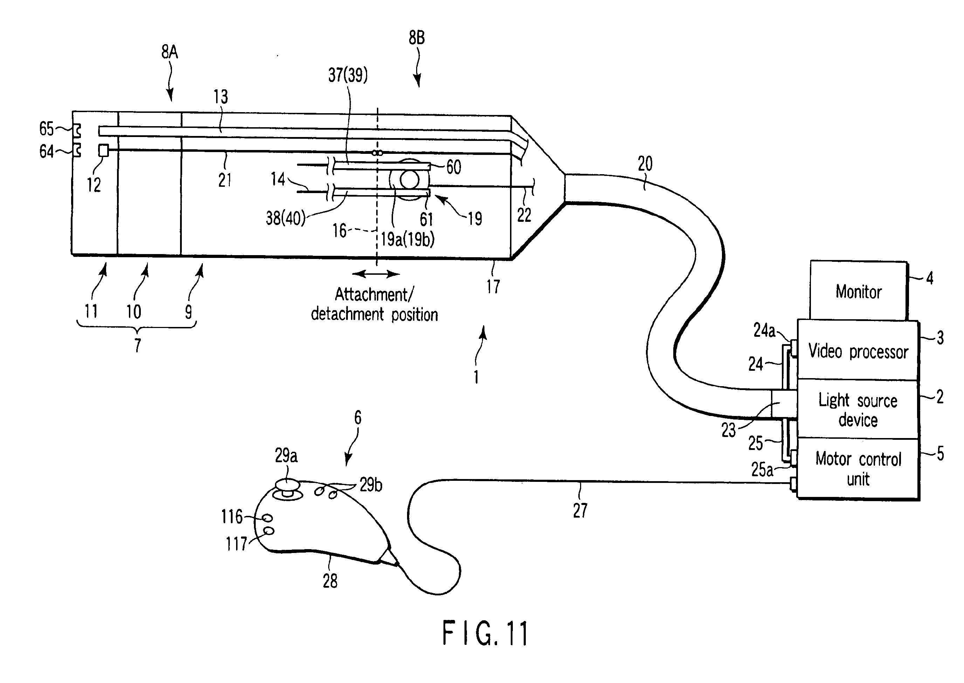

FIG. 11 schematically shows the structure of the entire system of a detachable-type endoscope 1 according to a fourth embodiment of the present invention. In this embodiment, the structure of the scope section 8A of the detachable-type endoscope 1 according to the first embodiment (see FIG. 1 to FIG. 6B) is altered as described below. The other structural parts are the same as those of the detachable-type endoscope 1 according to the first embodiment. The parts common to those of the detachable-type endoscope 1 according to the first embodiment are denoted by like reference numerals, and a description thereof is omitted.

Specifically, in this embodiment, conduits in the insertion section 7 of the scope section 8A in the first embodiment, such as the therapeutic device insertion conduit 112, water feed conduit 113 and air feed conduit 114, are dispensed with.

The handpiece 28 of the operation unit 6 is provided with a joystick 29a for remotely bend-operating the bending section 10 and other remote switches 29b.

By operating the joystick 29a of the handpiece 28, the bending section 10 is remotely bend-operated. In addition, the endoscope operations corresponding to the functions of the remote switches 29b are performed by operating the remote switches 29b.

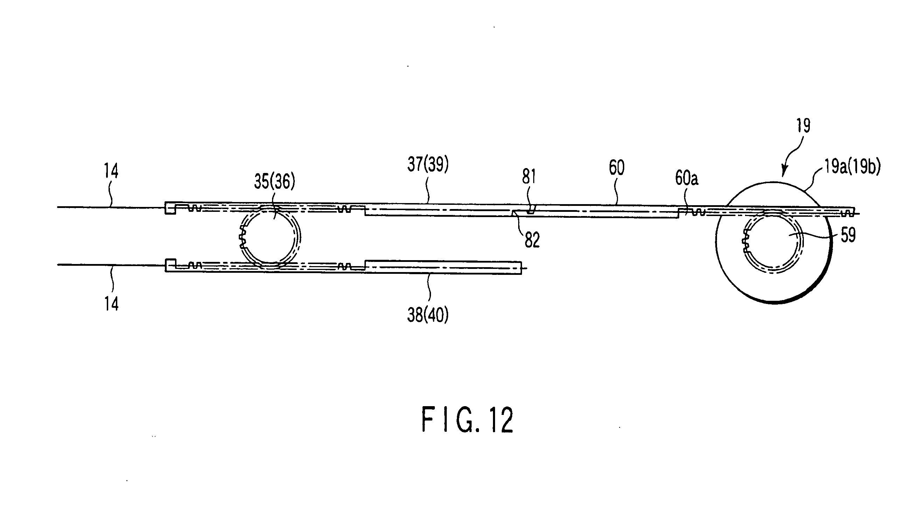

FIG. 12 shows a fifth embodiment of the present invention. In this embodiment, the structure of the coupling section between the coupling end section 16 of the large-diameter section 15 of the scope section 8A and the coupling end section 18 of the driving source unit 8B in the detachable-type endoscope 1 according to the first embodiment (see FIG. 1 to FIG. 6B) is altered as described below. The other structural parts are the same as those of the detachable-type endoscope 1 according to the first embodiment. The parts common to those of the detachable-type endoscope 1 according to the first embodiment are denoted by like reference numerals, and a description thereof is omitted.

Specifically, in the structure of the first embodiment, the pair of driving shafts 60 and 61 are disposed to be opposed in parallel on both sides of the driving pinion 59 that is provided on the rotational shaft of the two (first and second) driving motors 19a, 19b which are built in the driving source unit 8B. Instead of this structure, in the present embodiment, the driving shafts 60 are disposed only on one side of the driving pinion 59 that is provided on the rotational shaft of the first and second driving motors 19a, 19b.

In the present embodiment, a recess-shaped stepped portion (engaging portion) 81 is provided at a distal-end-side shaft end portion of each driving shaft 60. Further, a stepped notch portion 82, which corresponds to the stepped portion 81 of each driving shaft 60, is provided at a shaft end portion of each of the two working shafts 37 and 39 of the four working shafts 37 to 40 that are projectingly provided at the coupling end section 16 on the scope section 8A side. When the driving source unit 8B and the scope unit 8A are coupled, the stepped portion 81 of each driving shaft 60 is detachably engaged with the notch portion 82 of each working shaft 37, 39. Thereby, a removal prevention section is formed which prevents removal of the working shaft 37, 39 from the driving shaft 60.

Next, the operation of the above-described structure is described. In the detachable-type endoscope 1 of this embodiment, prior to the coupling between the driving source unit 8B and the scope section 8A, the notch portion 82 of each working shaft 37, 39 and the stepped portion 81 of each driving shaft 60 are held in the separated state.

When the driving source unit 8B and the scope section 8A are to be coupled, as shown in FIG. 12, the notch portion 82 of each working shaft 37, 39 and the stepped portion 81 of each driving shaft 60 are detachably engaged. At this time, hook-like engagement is effected between the notch portion 82 of each working shaft 37, 39 and the stepped portion 81 of each driving shaft 60. Therefore, in the state in which the notch portion 82 of each working shaft 37, 39 and the stepped portion 81 of each driving shaft 60 are engaged, the engagement therebetween is not released even if the working shaft 37, 39 and the driving shaft 60 are moved in the axial direction.

According to this structure, when the driving shaft 60 moves toward the scope section 8A, an end portion of the driving shaft 60 abuts on the end portion of the working shaft 37, 39 on the driving source unit 8B side, and moves the working shaft 37, 39 toward the distal end of the scope section 8A. In accordance with the movement of the working shaft 37, 39 at this time, the other working shaft 38, 40 retreats in a direction opposite to the direction of movement of the working shaft 37, 39 via the pinion gear 35, 36. At this time, the wire 14, which is positioned on the lower side in FIG. 12, is pulled by the retreat movement of the working shaft 38, 40, and the bending section 10 is bent, for example, upward (or leftward).

When the driving shaft 60 moves toward the driving source unit 8B, the end face of the notch portion 82 of the working shaft 37, 39, which is engaged with the stepped portion 81 of the driving shaft 60, pulls the working shaft 37, 39, and moves the working shaft 37, 39 toward the driving source unit 8B. At this time, the wire 14, which is positioned on the upper side in FIG. 12, is pulled by the retreat movement of the working shaft 37, 39, and the bending section 10 is bent, for example, downward (or rightward). In accordance with the movement of the working shaft 37, 39 at this time, the other working shaft 38, 40 advances in a direction opposite to the direction of movement of the working shaft 37, 39 via the pinion gear 35, 36. The wire 14, which is positioned on the lower side in FIG. 12 and is fixed to the working shaft 38, 40 that is pushed forward (to the insertion section 7 side), is pulled by the bending section 10, and drawn to the insertion section 7 side.

The following advantageous effects can be obtained by the above-described structure. In the present embodiment, the structure of the coupling section between the coupling end section 16 of the large-diameter section 15 of the scope section 8A and the coupling end section 18 of the driving source unit 8B in the detachable-type endoscope 1 according to the first embodiment is altered as follows. Specifically, the driving shafts 60 are disposed only on one side of the driving pinion 59 that is provided on the rotational shaft of the first and second driving motors 19a, 19b. The stepped portion 81 is provided at the distal-end-side shaft end portion of each driving shaft 60. The stepped notch portion 82 is provided at the shaft end portion of each of the two working shafts 37 and 39 of the four working shafts 37 to 40 that are projectingly provided at the coupling end section 16 on the scope section 8A side. Thereby, when the driving source unit 8B and the scope unit 8A are coupled, the notch portion 82 of each working shaft 37, 39 is detachably engaged with the stepped portion 81 of each driving shaft 60, as shown in FIG. 12. Thus, the removal prevention section is formed which prevents removal of the working shaft 37, 39 from the driving shaft 60. Therefore, it is possible to provide the detachable-type endoscope 1 in which the number of parts is smaller, the structure is simpler and the cost is lower than in the structure of the first embodiment (see FIG. 3).

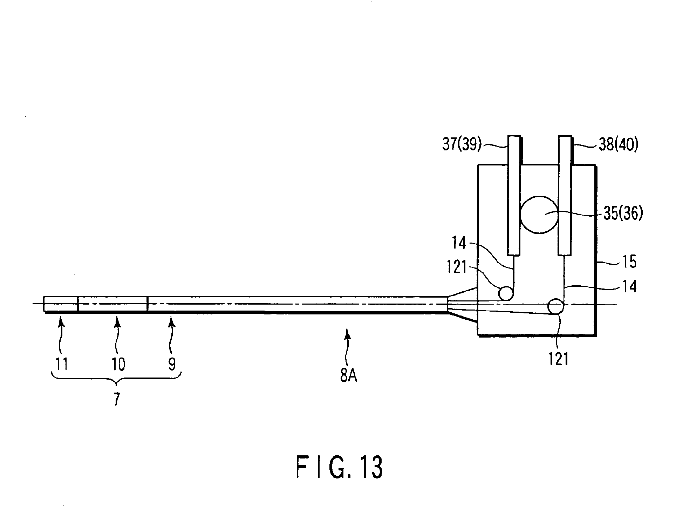

FIG. 13 shows a sixth embodiment of the present invention. In this embodiment, the structure of the driving force transmission means 34, which is built in the large-diameter section 15 of the scope section 8A of the detachable-type endoscope 1 according to the first embodiment (see FIG. 1 to FIG. 6B), is altered as described below. The other structural parts are the same as those of the detachable-type endoscope 1 according to the first embodiment. The parts common to those of the detachable-type endoscope 1 according to the first embodiment are denoted by like reference numerals, and a description thereof is omitted.

Specifically, the driving force transmission means 34 of the present embodiment is provided with pulleys 121 which bend the directions of the wires 14, which extend in the large-diameter section 15, approximately at right angles. In the present embodiment, the four working shafts 37 to 40 of the driving force transmission means 34, which is assembled in the large-diameter section 15 of the scope section 8A, are made to extend in a direction substantially perpendicular to the axial direction of the insertion section 7 of the scope section 8A.

Needless to say, the present invention is not limited to the above-described embodiments, and various modifications may be made without departing from the spirit of the invention.

Next, other characteristic technical items of the present invention are described below.

### NOTE

(Item 1) An endoscope apparatus in which an operation section for performing a bend operation and a scope section, which includes a distal end section, a bending section, a rigid or flexible insertion section and a coupling section for coupling to the operation section, are detachably coupled, characterized in that a pair of shaft members, which have proximal end portions connected to wires that bend the distal end section in a predetermined direction, are moved in mutually opposite directions at a time of the bend operation.
(Item 2) The endoscope apparatus according to item 1, characterized in that the pair of shaft members, which have the proximal end portions connected to the wires, are provided with racks that are meshed with a pinion, which is rotatably supported in the coupling section, in a mutually opposed manner.
(Item 3) The endoscope apparatus according to item 1, characterized in that a guide member, which guides the pair of shaft members in a direction of movement, is provided.
(Item 4) The endoscope apparatus characterized in that the guide member according to item 3 is constituted by a guide member which is configured such that flat portions are provided along side surfaces of the rack portions provided on the pair of shaft members and the guide member slidably guides these parts.
(Item 5) The endoscope apparatus characterized in that the guide member according to item 3 is constituted by a guide member which slidably guides opposite surfaces to the rack portions provided on the pair of shaft members.
(Item 6) The endoscope apparatus characterized in that the guide member according to item 3 is constituted by a guide member which is configured such that slits which extend in a direction of movement are provided on a side opposite to the racks of the pair of shaft members, and projection portions which are engaged in the slits are provided.
(Item 7) The endoscope apparatus characterized in that the guide member according to item 3 is constituted by a guide member which is configured such that the pair of shaft members are provided with end portions projecting to a pinion side from the rack portions, and the end portions are slidably guided.
(Item 8) An endoscope characterized by comprising an insertion section which includes a bend section composed by coupling a plurality of bend portions and is insertable in a body cavity; a proximal section which is provided on a proximal end side of the insertion section; a main body section which is attachable/detachable to/from the proximal section; operation means which is provided in the main body section and includes a pair of advancement/retreat members that linearly move in mutually opposite directions; driven members which are provided in the proximal section, are paired with the advancement/retreat members, and are pushed when the advancement/retreat members move in a predetermined direction; reversing means which is provided in the proximal section, transmits a driving force of one of the driven members, which moves in accordance with the advancement/retreat members, to the other driven member, and reverses a direction of the movement of the other driven member, relative to said one of the driven members, which moves in accordance with the advancement/retreat members; and wires which have distal end portions connected to the bending section, have proximal end sides connected to the driven members, and bend the bending section in interlock with the operation of the driven members.
(Item 9) The endoscope according to claim 8, characterized in that rack portions are formed on the driven members, and a pinion, which is meshed with the rack portions and is rotated in accordance with advancement/retreat of the rack portions, is formed in the reversing means.
(Item 10) An endoscope characterized by comprising an insertion section which is insertable in a body cavity; a bend section which is disposed on a distal end side of the insertion section and is constituted by coupling a plurality of bend pieces; a pair of wires for a bend operation of the bending section, the wires having distal end portions connected to the bending section and having proximal end portions extending to a proximal end side of the insertion section; a coupling section provided on the proximal end side of the insertion section; a pair of working shafts which are disposed in the coupling section substantially in parallel to an axial direction of the insertion section, are connected to the proximal end portions of the wires, and linearly move in mutually opposite directions; reversing means which is interposed between the pair of working shafts and transmits movement of one of the working shaft to the other working shaft, with the direction of the movement being reversed; a driving source unit which is detachably coupled to the coupling section and includes driving force generating means for generating a driving force for bending the bending section; and operation means which is provided in the driving source unit, includes a pair of driving shafts which are linearly moved in mutually opposite directions by the driving force from the driving force generating means, and linearly moves one of the working shafts in accordance with the movement of one of the driving shafts which is linearly moved forward by the driving force from the driving force generating means when the driving source unit and the coupling section are coupled.
(Item 11) The endoscope according to claim 11, characterized in that the working shafts have rack portions, the reversing means includes a pinion gear that is meshed with the rack portions and rotates in accordance with advancement/retreat of the rack portions.

### Industrial Applicability

The present invention is effective in a technical field in which use is made of an endoscope of a driving source unit detachable type, wherein a driving source unit incorporating driving force generating means for bend-operating a bending section, which is disposed on a distal end side of an insertion section of the endoscope, is detachably coupled to a proximal section of the insertion section via an attachment/detachment section, and in a technical field of manufacture of this endoscope.

## Claims

1. An endoscope comprising:
an insertion section (7) which includes a distal end portion and a proximal end portion and is insertable in a body cavity, the insertion section (7) including a bending section (10);
a coupling section (15) provided on a side of the proximal end portion of the insertion section (7);
a pair of working shafts (37, 38) provided at the coupling section (15);
a driving source unit (8B) which is detachably coupled to the coupling section (15) and includes driving force generating means (19) adapted for generating a driving force for bending the bending section (10);
operation means (59-61) which is provided in the driving source unit (8B), includes a driving shaft (60, 61) that is adapted to linearly move on the basis of the driving force generated by the driving force generating means (19), and moves at least one of the working shafts (37, 38) at a time of coupling to the coupling section (15);
reversing means (35) which is provided in the coupling section (15) and which is adapted to transmit movement of one of the working shafts (37, 38), which is moved by the driving shaft, to the other of the working shafts (37, 38) in a direction opposite to a direction of the movement; and
bend operation wires (14) each having a distal end portion and a proximal end portion, the distal end portions of the bend operation wires (14) being connected to the bending section (10), the proximal end portions of the bend operation wires (14) being connected to the working shafts (37, 38) which are mutually reversely moved, the bend operation wires (14) adapted for bend-operating the bending section (10) in accordance with the movement of the working shafts (37, 38),
**characterized in that**
the bending section (10) is constituted by coupling a plurality of bend pieces;
the reversing means (35) is composed of a pinion gear (35), and
the working shafts (37, 38) have rack portions (37a, 38a) which are meshed with the pinion gear (35) in a mutually opposed manner.

2. The endoscope according to claim 1, wherein the driving source unit (8B) includes a pair of said driving shafts (60, 61) which move the pair of working shafts (37, 38), respectively.

3. The endoscope according to claim 1, wherein
the insertion section (7) includes a rigid or flexible insertion tube section (9) having a distal end portion and a proximal end portion,
the bending section (10) is configured to be bend-operable in four directions, that is, an upward direction, a downward direction, a leftward direction and a rightward direction,
the bend operation wires (14) comprise two wires (14) for an up-and-down bend operation, which bend-operates the bending section (10) in an up-and-down direction, and two wires (14) for a right-and-left bend operation, which bend-operates the bending section (10) in a right-and-left direction,
the coupling section (15) includes two sets of said pair of working shafts (37, 38), and reversing means (35) which is interposed between the pair of working shafts (37, 38) of each of the two sets, and
the driving source unit (8B) includes:
driving force generating means (19) for an up-and-down direction and driving force generating means (19) for a right-and-left direction, which generate driving forces for bending the bending section (10) in the up-and-down direction and the right-and-left direction, respectively; and
wherein the operation means (59,61) includes driving shafts (60, 61) that are adapted to linearly move on the basis of the driving forces generated by the driving force generating means (19) for the up-and-down direction and the driving forces generated by the driving source generating means (19) for the right-and-left direction, and to move at least one of the working shafts (37, 38) of each of said sets at the time of coupling to the coupling section (15).

4. The endoscope according to claim 1, wherein
the coupling section (15) includes a base plate (33),
the pinion gear (35) is rotatably supported on the base plate (33), and
the base plate (33) includes a guide member (54-56, 71-73), which guides the working shafts (37, 38) in a direction of movement.

5. The endoscope according to claim 4, wherein the guide member (54,56, 71-73) includes a flat portion (71-72a) for slidable guiding along a side surface of the rack portion (37a, 38a) provided on the working shaft (37, 38).

6. The endoscope according to claim 4, wherein the guide member (54-56, 71-73) includes rotation prevention means (71 b, 72b, 73a, 73b) for preventing rotation of the working shaft (37, 38) about an axis thereof.

7. The endoscope according to claim 6, wherein the rotation prevention means is formed by a slit (37b, 38b, 39b and 40b) which is extendingly provided in the working shaft (37, 38) on a side opposite to the rack portion (37a, 38a) along the direction of movement of the working shaft (37, 38), and a projection portion (54a, 54b) which is projectingly provided on the guide member (54-56, 71-73) and is engaged in the slit (37b, 38b, 39b and 40b).

## Patentansprüche

1. Endoskop, umfassend:
einen Einführbereich (7), der einen distalen Endabschnitt und einen proximalen Endabschnitt aufweist und in eine Körperhöhle einführbar ist, wobei der Einführbereich (7) einen Biegebereich (10) aufweist;
einen auf einer Seite des proximalen Endabschnitts des Einführbereichs (7) vorgesehenen Kupplungsbereich (15);
ein Paar von an dem Kupplungsbereich (15) vorgesehenen Arbeitsstäben (37, 38);
eine Antriebsquelleneinheit (8B), die lösbar mit dem Kupplungsbereich (15) gekoppelt ist und eine Antriebskrafterzeugungseinrichtung (19) aufweist, welche dazu ausgebildet ist, eine Antriebskraft zum Biegen des Biegebereichs (10) zu erzeugen;
eine in der Antriebsquelleneinheit (8B) vorgesehene Betätigungseinrichtung (59-61), die einen Antriebsstab (60, 61) aufweist, der so ausgebildet ist, dass er aufgrund der durch die Antriebskrafterzeugungseinrichtung (19) erzeugten Antriebskraft eine lineare Bewegung ausführt und während der mit dem Kupplungsbereich (15) gekoppelten Zeit wenigstens einen der Arbeitsstäbe (37, 38) bewegt;
eine in dem Kupplungsbereich (15) vorgesehene Umkehreinrichtung (35), ausgebildet zum Übertragen der Bewegung eines der Arbeitsstäbe (37, 38), der durch den Antriebsstab bewegt wird, auf den anderen der Arbeitsstäbe (37, 38) in einer der Bewegungsrichtung entgegengesetzten Richtung; und
Biegebetätigungsdrähte (14), die jeweils einen distalen Endabschnitt und einen proximalen Endabschnitt aufweisen, wobei die distalen Endabschnitte der Biegebetätigungsdrähte (14) mit dem Biegebereich (10) und die proximalen Endabschnitte der Biegebetätigungsdrähte (14) mit den in entgegengesetzte Richtungen bewegten Arbeitsstäben (37, 38) verbunden sind, und wobei die Biegebetätigungsdrähte (14) ausgebildet sind zum Biegebetätigen des Biegebereichs (10) entsprechend der Bewegung der Arbeitsstäbe (37, 38),
**dadurch gekennzeichnet, dass**
der Biegebereich (10) durch Kopplung mehrerer Biegeteile gebildet wird;
die Umkehreinrichtung (35) aus einem Ritzel (35) besteht, und
die Arbeitsstäbe (37, 38) Zahnstangenbereiche (37a, 38a) aufweisen, die sich einander gegenüberliegend mit dem Ritzel (35) in Eingriff befinden.

2. Endoskop nach Anspruch 1, wobei die Antriebsquelleneinheit (8B) ein Paar von Antriebsstäben (60, 61) aufweist, die das Paar von Arbeitsstäben (37, 38) bewegen.

3. Endoskop nach Anspruch 1, wobei
der Einführbereich (7) einen starren oder flexiblen Einführrohrabschnitt (9) mit einem distalen Endabschnitt und einem proximalen Endabschnitt aufweist,
der Biegebereich (10) so ausgebildet ist, dass er in vier Richtungen, und zwar nach oben, nach unten, nach links und nach rechts, gebogen werden kann,
die Biegebetätigungsdrähte (14) zwei Drähte (14) für eine nach oben und nach unten gerichtete Biegebetätigung zum Biegebetätigen des Biegebereichs (10) nach oben und nach unten, und zwei Drähte (14) für eine nach rechts und nach links gerichtete Biegebetätigung zum Biegebetätigen des Biegebereichs (10) nach rechts und nach links umfassen,
der Kupplungsbereich (15) zwei Sets von Arbeitsstabpaaren (37, 38) und eine Umkehreinrichtung (35) aufweist, die zwischen dem Arbeitsstabpaar (37, 38) jedes der beiden Sets angeordnet ist, und wobei
die Antriebsquelleneinheit (8B) aufweist:
eine für eine Aufwärts- und Abwärtsrichtung ausgelegte Antriebskrafterzeugungseinrichtung (19) und eine für eine Rechts - und Linksrichtung ausgelegte Antriebskrafterzeugungseinrichtung (19) zum Erzeugen von Antriebskräften, um den Biegebereich (10) jeweils nach oben und nach unten sowie nach rechts und nach links zu biegen; und
wobei die Betätigungseinrichtung (59, 61) Antriebsstäbe (60, 61) aufweist, die ausgebildet sind, um eine lineare Bewegung auszuführen aufgrund der Antriebskräfte, die durch die für die Aufwärts- und Abwärtsrichtung ausgelegte Antriebskrafterzeugungseinrichtung (19) erzeugt werden, und der Antriebskräfte, die durch die für die Rechts- und Linksrichtung ausgelegte Antriebskrafterzeugungseinrichtung (19) erzeugt werden, und um wenigstens einen der Arbeitsstäbe (37, 38) jedes der Sets während der mit dem Kupplungsbereich (15) gekoppelten Zeit zu bewegen.

4. Endoskop nach Anspruch 1, wobei
der Kupplungsbereich (15) eine Grundplatte (33) aufweist,
das Ritzel (35) drehbar auf der Grundplatte (33) gelagert ist, und
die Grundplatte (33) ein Führungselement (54-56, 71-73) aufweist, das die Arbeitsstäbe (37, 38) in Bewegungsrichtung führt.

5. Endoskop nach Anspruch 4, wobei das Führungselement (54-56, 71-73) einen flachen Bereich (71-72a) aufweist zur Gleitführung entlang einer Seitenfläche des an dem Arbeitsstab (37, 38) vorgesehenen Zahnstangenbereichs (37a, 38a).

6. Endoskop nach Anspruch 4, wobei das Führungselement (54-56, 71-73) eine Drehsicherungseinrichtung (71b, 72b, 73a, 73b) aufweist, um eine Drehung des Arbeitsstabs (37, 38) um seine Achse zu verhindern.

7. Endoskop nach Anspruch 6, wobei die Drehsicherungseinrichtung durch einen Schlitz (37b, 38b, 39b and 40b), der sich in dem Arbeitsstab (37, 38) auf einer dem Zahnstangenbereich (37a, 38a) gegenüberliegenden Seite entlang der Bewegungsrichtung des Arbeitsstabs (37, 38) erstreckt, und einen vorspringenden Abschnitt (54a, 54b), der von dem Führungselement (54-56, 71-73) absteht und sich mit dem Schlitz (37b, 38b, 39b and 40b) in Eingriff befindet, gebildet wird.

## Revendications

1. Endoscope comprenant:
une section d'insertion (7) qui inclut une partie d'extrémité distale et une partie d'extrémité proximale et est insérable dans une cavité corporelle, la section d'insertion (7) incluant une section de courbure (10) ;
une section de couplage (15) prévue sur un côté de la partie d'extrémité proximale de la section d'insertion (7) ;
une paire d'arbres de travail (37, 38) prévue au niveau de la section de couplage (15) ;
une unité de source d'entraînement (8B) qui est couplée de façon détachable à la section de couplage (15) et inclut un moyen de génération de force d'entraînement (19) adapté à générer une force d'entraînement pour courber la section de courbure (10) ;
un moyen opérationnel (59-61) qui est prévu dans l'unité de source d'entraînement (8B) inclut un arbre d'entraînement (60, 61) qui est adapté à se déplacer linéairement sur la base de la force d'entraînement générée par le moyen de génération de force d'entraînement (19), et déplace au moins un des arbres de travail (37, 38) à un moment de couplage à la section de couplage (15) ;
un moyen d'inversion (35) qui est prévu dans la section de couplage (15) et qui est adapté à transmettre un mouvement de l'un des arbres de travail (37, 38), qui est déplacé par l'arbre d'entraînement, à l'autre des arbres de travail (37, 38) dans un sens opposé à un sens du mouvement ; et
des fils opérationnels de courbure (14) ayant chacun une partie d'extrémité distale et une partie d'extrémité proximale, les parties d'extrémité distale des fils opérationnels de courbure (14) étant connectées à la section de courbure (10), les parties d'extrémité proximale des fils opérationnels de courbure (14) étant connectées aux arbres de travail (37, 38) qui sont déplacés dans un sens mutuellement inverse, les fils opérationnels de courbure (14) adaptés à opérer en courbure la section de courbure (10) conformément au mouvement des arbres de travail (37, 38),
**caractérisé en ce que**
la section de courbure (10) est constituée en couplant une pluralité de pièces de courbure ;
le moyen d'inversion (35) est composé d'un pignon (35), et
les arbres de travail (37, 38) ont des parties de crémaillère (37a, 38a) qui sont engrenées avec le pignon (35) d'une manière mutuellement opposée.

2. Endoscope selon la revendication 1, dans lequel l'unité de source d'entraînement (8B) inclut une paire desdits arbres d'entraînement (60, 61) qui déplacent la paire d'arbres de travail (37, 38), respectivement.

3. Endoscope selon la revendication 1, dans lequel
la section d'insertion (7) inclut une section de tube d'insertion (9) rigide ou flexible ayant une partie d'extrémité distale et une partie d'extrémité proximale,
la section de courbure (10) est configurée pour être opérable en courbure dans quatre sens, à savoir un sens vers le haut, un sens vers le bas, un sens vers la gauche et un sens vers la droite,
les fils opérationnels de courbure (14) comprennent deux fils (14) pour une opération de courbure vers le haut et le bas, qui opèrent en courbure la section de courbure (10) dans un sens vers le haut et le bas, et deux fils (14) pour une opération de courbure vers la droite et la gauche, qui opèrent en courbure la section de courbure (10) dans un sens vers la droite et la gauche,
la section de couplage (15) inclut deux ensembles de ladite paire d'arbres de travail (37, 38), et un moyen d'inversion (35) qui est interposé entre la paire d'arbres de travail (37, 38) de chacun des deux ensembles, et
l'unité de source d'entraînement (8B) inclut:
un moyen de génération de force d'entraînement (19) pour un sens vers le haut et le bas et un moyen de génération de force d'entraînement (19) pour un sens vers la droite et la gauche, qui génèrent des forces d'entraînement pour courber la section de courbure (10) dans le sens vers le haut et le bas et le sens vers la droite et la gauche, respectivement ; et
dans lequel le moyen opérationnel (59, 61) inclut des arbres d'entraînement (60, 61) qui sont adaptés à se déplacer linéairement sur la base des forces d'entraînement générées par le moyen de génération de force d'entraînement (19) pour le sens vers le haut et le bas et des forces d'entraînement générées par le moyen de génération de force d'entraînement (19) pour le sens vers la droite et la gauche, et à déplacer au moins un des arbres de travail (37, 38) de chacun desdits ensembles au moment du couplage à la section de couplage (15).

4. Endoscope selon la revendication 1, dans lequel
la section de couplage (15) inclut une plaque de base (33),
le pignon (35) est supporté de façon rotative sur la plaque de base (33), et
la plaque de base (33) inclut un élément de guidage (54-56, 71-73) qui guide les arbres de travail (37, 38) dans un sens de mouvement.

5. Endoscope selon la revendication 4, dans lequel l'élément de guidage (54-56, 71-73) inclut une partie plate (71-72a) pour un guidage en coulissement le long d'une surface latérale de la partie de crémaillère (37a, 38a) prévue sur l'arbre de travail (37, 38).

6. Endoscope selon la revendication 4, dans lequel l'élément de guidage (54-56, 71-73) inclut un moyen de prévention de rotation (71b, 72b, 73a, 73b) pour prévenir une rotation de l'arbre de travail (37, 38) autour d'un axe de celui-ci.

7. Endoscope selon la revendication 6, dans lequel le moyen de prévention de rotation est formé par une fente (37b, 38b, 39b et 40b) qui est prévue en extension dans l'arbre de travail (37, 38) sur un côté opposé à la partie de crémaillère (37a, 38a) dans le sens de mouvement de l'arbre de travail (37, 38), et une partie de saillie (54a, 54b) qui est prévue en projection sur l'élément de guidage (54-56, 71-73) et est engagée dans la fente (37b, 38b, 39b et 40b).
